# EUROPÄISCHE PATENTSCHRIFT

(11) **EP 2 862 849 B1**
(45) Veröffentlichungstag und Bekanntmachung des Hinweises auf die Patenterteilung: **24.05.2017**
(21) Anmeldenummer: 14185987.6
(22) Anmeldetag: 23.09.2014
(51) Int. Cl.: B01J 29/46, C25B 1/04, C25B 15/08

(54) **Verfahren zur Umwandlung von CO2 zu Kohlenwasserstoffen**
Method for the conversion of CO2 to hydrocarbons
Procédé de conversion de CO2 en hydrocarbures

(30) Priorität: 23.09.2013 DE 102013110470; 23.09.2013 DE 102013022290
(43) Veröffentlichungstag der Anmeldung: 22.04.2015
(73) Patentinhaber: i² Gesellschaft für Innovation mbH, 34497 Korbach (DE)
(72) Erfinder: Koehne, Tim, 28215 Bremen (DE); Böhm, Raphael, 28209 Bremen (DE)
(74) Vertreter: Michalski Hüttermann & Partner Patentanwälte mbB

(56) Entgegenhaltungen:
- EP-A1- 0 355 229
- JP-A- H0 352 825
- US-A- 5 140 049

## Beschreibung

Die vorliegende Erfindung betrifft ein Verfahren zur Herstellung von flüssigen Kohlenwasserstoffen aus CO₂. Offenbart wird weiterhin eine Anlage zur Anwendung des Verfahrens zur Umwandlung von CO₂ in Kohlenwasserstoffe. Dadurch können CO₂-Emissionen in Industrie und Technik gesenkt werden und auf einfache Art und Weise Kohlenwasserstoffe generiert werden.

Etwa 40 Prozent der weltweiten CO₂-Emissionen werden im Wesentlichen durch Verbrennungsprozesse von fossilen Energieträgern in Kraftwerken erzeugt. Es besteht daher ein großer Bedarf an neuen technischen und nachhaltigen Lösungsansätzen, diese Emissionen deutlich zu reduzieren. Darüber hinaus besteht ebenfalls ein erheblicher Bedarf an Kohlenwasserstoffen, sowohl als Energieträger als auch als Ressource für chemische Prozesse. Unabhängig davon existieren Lösungsansätze, die teilweise temporär im Überschuss vorhandene durch regenerative Prozesse wie etwa Windkraft und Wasserkraft erzeugte elektrische Energie in flüssiger oder gasförmiger Form, insbesondere als Brennstoff, zu speichern. Diese Lösungsansätze sind im Stand der Technik unter dem Begriff "power to liquids" bekannt. Ebenfalls bekannt ist die Umwandlung von Elektrizität mittels Elektrolyse in gasförmigen Wasserstoff, welcher sowohl als Energieträger beispielsweise in Brennstoffzellen als auch als Grundstoff für chemische Prozesse dienen kann.

Diese Prozesse, insbesondere die power to liquid-Prozesse, können insbesondere dann einen notwendigen Beitrag zum Wandel der zukünftigen Energie- und Rohstoffversorgung leisten, wenn elektrische Energie aus regenerativen Prozessen verwendet wird, um aus in Abgasen von Industrieanlagen und Kraftwerken enthaltenes CO und CO₂ in flüssige Kohlenwasserstoffe umzuwandeln. Ein Verfahren zur Umwandlung von CO in flüssige Kohlenwasserstoffe, die Fischer-Tropsch-Synthese, ist seit langem bekannt. Das dieser Reaktion zugrunde liegende Gleichgewicht lässt sich durch die Formel (1) darstellen:

(1) *n* CO + *2n* H₂ ↔ CₙH₂ₙ₊₂ + n H₂O

Weiterhin ist bekannt, dass sich CO und CO₂ miteinander mit H₂ und H₂O im Gleichgewicht stehen, der sogenannten Wasser-Gas-Shift-Reaktion der Formel (2):

(2) H₂ + CO₂ ↔ H₂O + CO; ΔG⁰_{(298 K)} 28 KJ mol ⁻¹

Um CO₂ in ein für das Fischer-Tropsch-Verfahren nutzbares CO umzuwandeln, ist ein erheblicher Energieeintrag und Wasserstoffgas notwendig. Auch wenn dieser Prozess katalytisch durchgeführt wird, ist eine vom Fischer-Tropsch-Verfahren separate Prozessstufe notwendig.

Das schon 1925 entwickelte Fischer-Tropsch-Verfahren umfasst die katalytische Umsetzung von CO mit Wasserstoff zu Kohlenwasserstoffen. Als Katalysatoren kommen gemeinhin auf Eisen basierende Verbindungen zum Einsatz. Dieses Verfahren wird auch heute noch zur sogenannten Kohleverflüssigung eingesetzt, dabei werden die oben genannten Gleichgewichte dahingehend ausgenutzt, dass Kohle zu Kohlenmonoxid umgesetzt wird, das dann mittels des Fischer-Tropsch-Verfahrens mit Wasserstoff zu flüssigen Kohlenwasserstoffen umgesetzt wird.

Zusammengefasst besteht ein erheblicher Bedarf an einer Reduktion der CO₂-Emissionen von großtechnischen Anlagen, wie insbesondere Kraftwerken und Industrieanlagen. Es besteht ebenfalls ein erheblicher Bedarf an flüssigen Kohlenwasserstoffen als Energieträger und Rohstoffquelle für die chemische Synthese. Ein großer Bedarf existiert ebenfalls an Methoden zur Speicherung der durch regenerativen Prozesse erzeugte elektrischen Energie. In diesem Zusammenhang besteht ein Bedarf an effizienteren power to fuels-/power to liquid-Verfahren, insbesondere an effizienteren Fischer-Tropsch-Verfahren.JP H03-52825 offenbart Umwandlung von Kohlendioxid und Wasserstoff zu Propan. Es wurde gefunden, dass durch die Verwendung eines Katalysators, umfassend einen Zeolith-Anteil, und einen Fe-Katalysator-Anteil, ein Verfahren möglich wird, in dem in einem Verfahrensschritt die Umsetzung von CO₂ mit H₂ zu flüssigen Kohlenwasserstoffen gelingt.

Die vorliegende Erfindung stellt daher folgendes bereit:
Ein Verfahren zur Umwandlung von CO₂ in flüssige Kohlenwasserstoffe, umfassend die Schritte
   (i) Bereitstellen eines Gasstroms, umfassend CO₂ und Wasserstoffgas,
   (ii) Kontaktieren des Gasstromes und mindestens einem Katalysator, umfassend
      (a) einen Zeolith-Anteil, und
      (b) einen Fe-Katalysator-Anteil, umfassend Eisen im Bereich von 20 bis 200 Gewichtsteilen, einen Al₂O₃-Träger im Bereich von 5 bis 100 Gewichtsteilen, Kupfer im Bereich von 1 bis 40 Gewichtsteilen und mindestens ein Alkalimetall und/oder ein Erdalkalimetall im Bereich von 1 bis 40 Gewichtsteilen,
         wobei die Anteile (a) und (b) in einer physikalischen Mischung in einem Verhältnis im Bereich von 10:1 bis 4:1 vorliegen,
         bei einer Temperatur im Bereich von 250 bis 450 °C und einem Druck im Bereich von 0,1 bis 10 MPa,
dabei Umwandeln des CO₂ in flüssige Kohlenwasserstoffe.

Durch dieses Verfahren ist es möglich, CO₂ aus einem Abgasstrom wie insbesondere eines Kraftwerks, einer Industrieanlage wie insbesondere einer Anlage zur Metallveredelung oder einer Härterei, oder einem Schiffsaggregat direkt in flüssige Kohlenwasserstoffe, umzuwandeln. Verglichen mit der klassischen Fischer-Tropsch-Synthese benötigt das erfindungsgemäße Verfahren nicht die Zufuhr von CO. Damit sind ebenfalls die Herstellung von CO mittels der sehr energieaufwändigen Kohlevergasung und der Abbau der Kohle dafür überflüssig geworden. Mit dem erfindungsgemäßen Katalysator und dem erfindungsgemäßen Verfahren werden also nicht nur die Ressourcen in Form von fossilen Brennstoffen wie etwa Kohle, Erdgas oder Erdöl geschont, sondern darüber hinaus mittels der direkten Nutzung von CO₂ eine völlig neue Ressource in großem Maßstab erschlossen.

Dabei wird insbesondere auch die Erzeugung von bei Standardbedingungen flüssiger Kohlenwasserstoffe ermöglicht. Dieses ist zur Speicherung von Energie besonders vorteilhaft. So liegt die Energiedichte von beispielsweise Diesel bei etwa 9500 kWh/m³, die von Methan hingegen von lediglich etwa 10 kWh/m³. Somit können auch große Energiemengen effizient gelagert und transportiert werden. Eine energieaufwändige Komprimierung, wie sie beispielsweise bei Methan notwendig sein kann, entfällt ebenfalls.

In einer bevorzugten Ausführungsform der vorliegenden Erfindung umfassen die Kohlenwasserstoffe bei Standardbedingungen flüssige Kohlenwasserstoffe. Dieses sind bevorzugt Gemische aus flüssigen Kohlenwasserstoffen. Kohlenwasserstoffe, die unter diese Definition fallen, können aliphatische, verzweigte und aromatische Kohlenwasserstoffe sein, insbesondere diejenigen, die sich unter den Begriffen Benzin, Diesel oder Paraffinöle subsummieren lassen. Dabei können sich die Mischungsverhältnisse der Kohlenwasserstoffe als auch die Art der Kohlenwasserstoffe durch die jeweiligen Betriebsparameter unterscheiden.

Der CO₂ umfassende Gasstrom der vorliegenden Erfindung kann in besonders vorteilhafter Weise auch direkt der Abgasstrom eines Kraftwerkes oder einer Industrieanlage sein. Es ist dabei bevorzugt, dass der Abgasstrom durch dem Fachmann bekannte und übliche Verfahren der Rohgasaufbereitung vorgereinigt wird, um insbesondere feste Bestandteile wie Ruße oder flüssige Bestandteile wie Wasser und darüber hinaus noch Sauerstoff zumindest zu einem Großteil zu entfernen. Der CO₂ umfassende Gasstrom kann, wenn er ein solcher vorgereinigter Abgasstrom ist, bevorzugt auch Stickstoffgas, Wasserdampf und/oder CO enthalten. Ganz besonders bevorzugt enthält der CO₂ umfassende Gasstrom Stickstoffgas und kann einen Anteil CO enthalten, und insbesondere bevorzugt wurde dem Abgasstrom zusätzlich zu dem schon vorhandenen Stickstoff kein weiteres Stickstoffgas zugesetzt. Das hat den Vorteil, dass dadurch die Nachhaltigkeit des Verfahrens im Sinne einer Energieeinsparung verbessert wird, da kein Stickstoffgas separat erzeugt werden muss und so Energie eingespart werden kann.

Die Abtrennung von Rußen, Wasserdampf und Sauerstoffgas kann nach üblichen und dem Fachmann bekannten Verfahren durchgeführt werden. Die Sauerstoffabtrennung kann beispielsweise mittels einer Druckwechsel-Adsorption (engl. Pressure swing adsporption, PSA) erfolgen. Dabei kann der vorzugsweise von Rußanteilen vorgereinigte Gasstrom unter erhöhtem Druck von beispielsweise 0,6 bis 1 MPa in einen Festbettreaktor, der mit einem Adsorbens wie beispielsweise Aktivkohle gefüllt ist eingeleitet werden und so das Adsorbens durchströmt werden, um den Sauerstoff zu adsorbieren. Am Ausgang des Bettes des Festbettreaktors tritt der nun vom Sauerstoff befreite Stickstoffgasstrom aus. Nach der Sättigung des Adsorbens mit Sauerstoff kann durch Druckabsenkung der Sauerstoff wieder aus dem Adsorbens erhalten werden und so letzteres regeneriert werden.

Dabei ist es ganz besonders bevorzugt, wenn der CO₂ umfassende Gasstrom, insbesondere der Abgasstrom eines Kraftwerks oder einer Industrieanlage, zusätzlich ein Inertgas enthält. Ein Inertgas im Sinne der vorliegenden Erfindung ist ein Gas, welches an der durch den erfindungsgemäßen Katalysator katalysierten Umwandlung von CO₂ in Kohlenwasserstoffe nicht teilnimmt und darüber hinaus den Katalysator nicht vergiftet. Das Inertgas ist ausgewählt aus der Gruppe bestehend aus Stickstoff, Argon, Neon und Helium. Besonders bevorzugt ist dabei Stickstoff. Das Zufügen eines Inertgases hat den Vorteil, dass auf diese Art und Weise sich besonders effektiv ansonsten nachteilige "hot spots" im Katalysator vermeiden lassen. Dabei kann das Inertgas, insbesondere Stickstoff, dem CO₂ umfassenden Gasstrom unmittelbar vor dem Inkontaktbringen mit dem erfindungsgemäßen Katalysator beigefügt werden. Alternativ kann eine Mischung aus Inertgas, insbesondere Stickstoff, CO₂ und H₂ hergestellt werden und als Gasstrom verwendet werden. Bei der Verwendung von Stickstoffgas bietet sich ein weiterer Vorteil: Das Stickstoffgas ist üblicherweise in Abgasströmen von Kraftwerken etc. schon enthalten, eine ansonsten gesonderte Beimengung von Stickstoffgas und/oder anderen Schutzgasen entfällt daher. Dieses ist dahingehend besonders vorteilhaft, da Schutzgase wie Stickstoff oder Edelgase wie Argon einen nicht unerheblichen Kostenfaktor darstellen können. In diesem Fall ist es bevorzugt, dass der CO₂- und Stickstoffgashaltige Abgasstrom beispielsweise eines mit der Verbrennung von fossilen Brennstoffen betriebenen Kraftwerkes direkt nach einem Reinigungsschritt zur Abtrennung von schwefelhaltigen Bestandteilen sowie Ruß und bevorzugt auch Sauerstoff mit Wasserstoffgas angereichert und als Synthesegas im erfindungsgemäßen Verfahren eingesetzt wird. Ist im von Wasserdampf, Schwefel bzw. schwefelhaltigen Verbindungen gereinigten CO₂-Synthesegas, insbesondere eines vorgereinigten Abgasstroms kein Stickstoffgas enthalten, so ist es bevorzugt, dem Synthesegas Stickstoffgas zuzusetzen. In einer ganz besonders bevorzugten Ausführungsform enthält der Abgasstrom des Kraftwerks, der Industrieanlage, des Verbrennungsmotors, der Biogasanlage, der Müllverbrennungsanlage oder der Pyrolyseanlage schon von sich die bevorzugten Anteile Stickstoffgas. Dadurch kann die gesonderte Zugabe von Stickstoffgas, das sonst gesondert unter Energieaufwand hergestellt wird, eingespart werden. Dadurch kann die Energiebilanz bzw. die CO₂-Bilanz der betreffenden Anlage verbessert werden. Vorzugsweise umfasst das Synthesegas in allen vorgenannten Fällen neben CO₂ und H₂ also N₂, bevorzugt N₂ in einem Anteil im Bereich von 0,1 bis 80 Volumen-% an der Gesamtmenge CO₂, H₂ und N₂, bevorzugt im Bereich von 30 bis 75 Volumen-%, und besonders bevorzugt im Bereich von 40 bis 65 Volumen-%.

Das Wasserstoffgas, welches in dem erfindungsgemäßen Verfahren verwendet wird, unterliegt keinen besonderen Beschränkungen. Es kann kommerziell erhältliches Wasserstoffgas oder direkt durch Elektrolyse hergestelltes Wasserstoffgas verwendet werden. Insbesondere beim Anschluss an Kraftwerke oder Industrieanlagen kann das Erzeugen von Wasserstoffgas durch Elektrolyse vorteilhaft sein, nämlich besonders dann, wenn überschüssige oder besonders günstige elektrische Energie und entsprechende Mengen Wasser verfügbar sind. In einer weiteren bevorzugten Ausführungsform der vorliegenden Erfindung umfasst das erfindungsgemäße Verfahren einen weiteren Schritt (ia) Erzeugen von Wasserstoffgas durch Elektrolyse. Die Gesamtenergiebilanz bzw. die Nachhaltigkeit des gesamten Prozesses lässt sich noch steigern, wenn die für die Erzeugung von Wasserstoffgas durch Elektrolyse im Schritt (ia) benötigte Elektrizität mittels Wasserkraft und/oder Windkraft und/oder einer Biogasanlage und/oder einer Pyrolyseanlage durchgeführt wird. Dieses ist besonders vorteilhaft, da die Erzeugung von elektrischer Energie durch Windkraft und Wasserkraft, insbesondere durch Windkraft, meistens nicht kontinuierlich erfolgen kann. So kann es insbesondere bei Windkraft vorkommen, dass bei erhöhtem Windaufkommen bei geringer Abnahme des Stromes durch Verbraucher oder erreichter Netzkapazität eine ansonsten ungenutzte überschüssige Menge an elektrischer Energie erzeugt wird. Die Verwendung von Windkraft und/oder Wasserkraft zur Erzeugung von Wasserstoffgas durch Elektrolyse führt daher in besonders vorteilhafter Weise zu einer Umwandlung von Energie, in diesem Fall vorteilhafterweise erneuerbarer Energie, in vielfältig speicherbare und nutzbare Kohlenwasserstoffe im Sinne eines "power to fuels"-Konzeptes.

In einer weiteren bevorzugten Ausführungsform des erfindungsgemäßen Verfahrens wird der Schritt (ii) bei einer Temperatur im Bereich von 300 bis 400 °C und einem Druck im Bereich von 0,5 bis 8 MPa, bevorzugt im Bereich von 0,8 bis 4 MPa durchgeführt. Offenbart wird auch eine Anlage zur Umwandlung von CO₂ in Kohlenwasserstoff in einem erfindungsgemäßen Verfahren, umfassend mindestens einen erfindungsgemäßen Katalysator.Die Anlage umfasst zusätzlich eine mit mittels Windkraft und/oder Wasserkraft erzeugter Elektrizität betriebene Elektrolyseeinrichtung. So liegen beispielsweise in Deutschland die Windkraftanlagen oftmals in Küstennähe oder sogar im küstennahen Bereich des Meeres (sogenannte "Off-shore-Anlagen"), während die stromverbrauchende Industrie und Städte viel weiter im Landesinneren liegen. In ähnlicher Weise verhält es sich mit Biogasanlagen und Pyrolyseanlagen, in denen organischer und/oder Kunststoffabfälle unter Druck und hoher Temperatur in Kohlenwasserstoffgase umgewandelt werden, die dann durch Verbrennung zur Energieerzeugung genutzt werden. Diese Anlagen liegen meist nicht dort, wo ein hoher Bedarf an Elektrizität besteht. Darüber hinaus ist die Situation bekannt, dass zu Zeiten hoher Stromerzeugung durch diese Anlagen der Bedarf an der erzeugten elektrischen Energie niedrig ist. Es besteht daher ein Bedarf einer effizienten Speicherung bzw. Zwischenspeicherung dieser Energie. Durch Umwandlung dieser zumindest zeitweise überschüssigen elektrischen Energie durch die bevorzugte Elektrolyseeinrichtung in Wasserstoffgas kann dieses besonders vorteilhaft im erfindungsgemäßen Verfahren mit dem CO₂-haltigen Gasstrom in bevorzugt flüssige Kohlenwasserstoffe umgewandelt werden. Dadurch wird zum einen eine sehr gute Energiespeichermöglichkeit erhalten, zum anderen kann dadurch die CO₂-Emission eines Kraftwerkes gesenkt werden. Zusätzlich wird durch die flüssigen Kohlenwasserstoffe ein wertvoller Energieträger und Synthesegrundstoff für die chemische Industrie erhalten.

Der Katalysator kann im erfindungsgemäßen Verfahren und in der Anlage bevorzugt in Form eines Wirbelbettes, Festbettreaktors und/oder einer Beschichtung eingesetzt werden. Unter einem Wirbelbett bzw. einer Wirbelschicht wird üblicherweise eine Schüttung von Feststoffpartikeln verstanden, welche durch eine aufwärtsgerichtete Strömung eines Fluids in einen fluidisierten Zustand versetzt wird. Im vorliegenden Fall bestehen die Feststoffpartikel aus dem erfindungsgemäßen Katalysator, das aufwärtsströmende Fluid umfasst den CO₂, Wasserstoffgas und gegebenfalls Stickstoffgas und/oder Biogas umfassenden Gasstrom. Wirbelbetten bzw. Wirbelschicht-Reaktoren und Methoden zu ihrer Dimensionierung, Ausgestaltung und Betrieb sind dem Fachmann aus dem Stand der Technik bekannt. Der Einsatz des erfindungsgemäßen Katalysators in Wirbelbetten bzw. Wirbelschichtreaktoren hat den Vorteil, dass es darin zu einem engen Kontakt des als Feststoffpartikel vorliegenden Katalysator mit dem Gasstrom und zu lebhaften Platzwechseln der einzelnen Partikel nach allen Richtungen kommt, was für die katalytisch betriebene Reaktionsführung gerade in größer dimensionierten Anlagen von erheblichen Vorteil ist. Dieses führt darüber hinaus zu einem guten Wärmetransport innerhalb der Anlage und zu einem guten Wärmeübergang zwischen Wirbelschicht und der Behälterwand bzw. eingebauten Wärmetauschern. Gleichzeitig sorgen der gute Wärmetransport und die (im Vergleich zu Gas) enorm hohe Wärmekapazität des Bettinventars für ein relativ homogenes Temperaturfeld in der Anlage. Unter einem Festbettreaktor wird ein Reaktor verstanden, in welchem der erfindungsgemäße Katalysator als feste Schüttung oder Packung vorliegt, die von einem Gasstrom durchströmt wird. Alternativ kann der erfindungsgemäße Katalysator in Form einer Beschichtung vorliegen, beispielsweise als Beschichtung eines röhrenförmigen Reaktors oder eines eine Vielzahl mit dem Katalysator beschichteten Röhren umfassenden Reaktors. Vorteilhaft dabei ist der geringe Innenwiderstand des Reaktors sowie die Möglichkeit der Wärmeabfuhr bzw. Wärmesteuerung der Anlage. In einer weiteren bevorzugten Ausführungsform des erfindungsgemäßen Verfahrens bzw. der erfindungsgemäßen Anlage kann der erfindungsgemäße Katalysator in Form eines mikrostrukturierten Reaktors vorliegen. Unter einem solchen mikrostrukturierten Reaktor oder auch Mikroreaktor wird gemeinhin ein kontinuierlich betriebener Reaktortyp mit Mikrokanälen verstanden, wie er beispielsweise in P. Watts, C. Wiles, Chem. Commun. 2007, 443-467; K. Schubert, J. Brandner, M. Fichtner, G. Linder, U. Schygulla, A. Wenka, Microscale Thermophysical Engineering (Taylor & Francis) 5, 1 beschrieben ist. Darin kann vorteilhafterweise der Katalysator entweder als Schüttung in die Kanäle oder als Beschichtung auf die Kanäle eingebracht sein. Durch die Kanäle wird der CO₂ umfassende Gasstrom geleitet. Die Verwendung des erfindungsgemäßen Katalysators in einem solchen Mikroreaktor im erfindungsgemäßen Verfahren führt zu einem besonders vorteilhaften schnellen Vermischen der Reaktanden, kurzen steuerbaren Verweilzeiten im Reaktor und damit einer sehr guten Regelbarkeit des Systems, einer hohen Betriebssicherheit sowie insbesondere einer besonders effektiven Wärmeübertragung und somit einer besonders guten Steuerbarkeit der Wärmeabfuhr. Die Anlagen können auch kleiner gebaut, mit geringeren Investitionen realisiert und effizienter betrieben werden. Ein weiterer Vorteil liegt in der Möglichkeit des numbering-up, d.h. in der Vergrößerung des Maßstabes durch einfaches Parallelschalten einzelner Mikroreaktor-Module. Damit lassen sich die ansonsten üblichen Probleme des scale-up bei üblichen Reaktortypen vermeiden. Der Fachmann kann daher die für ihn gewünschten Parameter des Verfahrens auf einem Mikroreaktormodul einstellen und braucht dieses dann nur numbering-up, also durch eine Erhöhung der Anzahl der Mikroreaktor-Module, auf einen größeren Maßstab zu übertragen.

Es ist bekannt, dass mittels Fe-Katalysatoren die Hydrierung von CO₂ zu Kohlenwasserstoffen gelingt. Allerdings sind die bekannten Verfahren hinsichtlich des Umsatzes, den Reaktionsbedingungen und der Selektivität bezüglich der Produkte noch verbesserungswürdig. Es wurde gefunden, dass durch eine bestimmte Kombination von Zusätzen zum Eisenanteil die Selektivität, die Handhabbarkeit und die Umsätze von CO₂ zu Kohlenwasserstoffen gesteigert werden kann. Erfindungsgemäß umfasst der Eisenanteil, d.h. der Fe-Anteil des Katalysators im erfindungsgemäßen Verfahren daher neben Eisen auch Kupfer und mindestens ein Alkalimetall und/oder Erdalkalimetall. Es wird angenommen, dass durch den Kupferanteil eine verbesserter Reduzierbarkeit des Eisenkatalysator-Anteils des erfindungsgemäßen Katalysators erhalten wird. Dieses hat den Effekt, dass zu dessen Aktivierung niedrigere Temperaturen als im Stand der Technik eingesetzt werden können. Eine niedrigere Reduktionstemperatur führt vorteilhafterweise zu einer höheren katalytischen Aktivität in der Hydrierung von CO₂ bzw. einer Fischer-Tropsch-Reaktion. Darüber hinaus wurde gefunden, dass durch eine Kombination von Eisen und Kupfer zusammen mit mindestens einem Alkalimetall und/oder einem Erdalkalimetall die Umsatzrate von CO₂ erhöht sowie eine Verschiebung der Produktverteilung zu einem höheren Olefin/Paraffin-Verhältnis erzielt werden kann. Es wird vermutet, dass sich eine KMH₄-Phase ausbildet, die möglicherweise als Wasserstoffreservoir fungiert und/oder den gasförmigen Wasserstoff während der Hydrierungsreaktion aktiviert, wobei M für Alkali- und/oder Erdalkalimetalle steht. Unter Alkalimetallen im Sinne der vorliegenden Erfindung werden insbesondere Lithium, Natrium und Kalium verstanden, unter den Erdalkalimetallen insbesondere Calcium, Rubidium und Caesium. Bevorzugt sind Alkalimetalle wie insbesondere Lithium, Natrium und Kalium und besonders bevorzugt ist Kalium. Bei Kalium ist die Erhöhung der Umsatzrate von CO₂ erhöht sowie die Verschiebung der Produktverteilung zu einem höheren Olefin/Paraffin-Verhältnis mit dem erfindungsgemäßen Katalysator am meisten ausgeprägt.

Aufgrund seiner besonderen Eignung ist in einer bevorzugten Ausführungsform daher der Katalysator des erfindungsgemäßen Verfahrens ein Katalysator zur katalytischen Hydrierung von Kohlenstoffoxiden, bevorzugt CO₂, in Kohlenwasserstoffe.

Dabei ist es bevorzugt, dass der Fe-Katalysator-Anteil im erfindungsgemäßen Verfahren b) Eisen im Bereich von 40 bis 180 Gewichtsteilen, besonders bevorzugt 70 bis 150 Gewichtsteilen, Al₂O₃ im Bereich von 10 bis 90 Gewichtsteilen, bevorzugt im Bereich von 20 bis 70 Gewichtsteilen, Kupfer im Bereich von 5 bis 30 Gewichtsteilen, bevorzugt im Bereich von 8 bis 20 Gewichtsteilen und mindestens ein Alkalimetall und/oder ein Erdalkalimetall im Bereich von 5 bis 30 Gewichtsteilen, bevorzugt im Bereich von 8 bis 20 Gewichtsteilen umfasst.

Unter den Zeolithen im Katalysator des erfindungsgemäßen Verfahrens werden dem Fachmann bekannte Zeolithe verstanden. Zeolithe umfassen Alumosilikat-Minerale, in denen AlO₄⁻ - und SiO₄-Tetraeder derart verbunden sind, dass sich eine mikroporöse Gerüststruktur ergibt.

Dabei ist der Zeolith des Zeolith-Anteils des Katalysators im erfindungsgemäßen Verfahren ausgewählt aus den Pentasil-Zeolithen. Zeolithe dieser Art haben den Vorteil, dass sie zum einen auch in größeren Mengen industriell verfügbar sind. Darüber hinaus zeigen diese Zeolithe in der erfindungsgemäßen Mischung mit dem Fe-Katalysator-Anteil eine synergistische Wirkung hinsichtlich der Katalyse der Hydrierung von CO₂ zu Kohlenwasserstoffverbindungen, insbesondere bezogen auf den Umsatz. Als besonders vorteilhaft haben sich Zeolithe des Typs ZSM-5, bevorzugt mit einem niedrigen Si/Al-Verhältnis erwiesen. Der Zeolith-Anteil im Katalysator hat eine umsatzerhöhende Wirkung, d.h. die Umsatzrate von CO₂ zu Kohlenwasserstoffen wird deutlich erhöht. Es wird dabei angenommen, dass der Zeolith das in der Reaktion gebildete Wasser aufnimmt bzw. durch Diffusionsprozesse aus der Reaktionszone und somit aus dem Reaktionsgleichgewicht entfernt und somit das Gleichgewicht auf die Produktseite gezogen wird. Vorstellbar ist ebenfalls, dass bei der Hydrierung von CO₂ bzw. CO teilweise Alkohole entstehen, die dann durch den Zeolith katalytisch dehydratisiert werden.

Durch die erfindungsgemäße Kombination aus (a) Fe-Katalysator gemäß der oben beschriebenen Zusammensetzung und (b) Zeolith innerhalb einer aktiven Zone wird eine besonders effiziente und selektive Umwandlung von CO₂ in Kohlenwasserstoffe unter besonders günstigen Reaktionsbedingungen ermöglicht. Das Vorliegen des Fe-Anteils des Katalysators und des Zeolith-Anteils des Katalysators bedeutet im Sinne der vorliegenden Erfindung, dass beide Katalysator-Funktionen als physikalisches Gemisch vorliegen. Der Zeolith-Anteil und der Fe-Anteil sind somit auf Mikroebene miteinander vermischt. Beide Katalysator-Funktionen liegen dabei als getrennte Cluster in direkter Nachbarschaft vor.

Das hat zur Folge, dass nun nicht mehr wie im Stand der Technik das beispielsweise im Abgasstrom eines Kraftwerkes enthaltene CO₂ in einem separaten, dem eigentlichen Fischer-Tropsch-Verfahren vorgelagerten Schritt in CO umgewandelt werden muss. Vielmehr findet die direkte Umwandlung von CO₂ in CO und die Umwandlung von CO und H₂ in die gewünschten Kohlenwasserstoffe innerhalb einer einzigen Zone und daher innerhalb eines einzigen Katalysator bzw. Reaktors statt. Es wird daher kein vorgelagerter Reaktor benötigt. Auf diese Weise wird Material gespart, was sich direkt in einer Verkleinerung einer entsprechenden Anlage bemerkbar macht. Darüber hinaus wird eine erhöhte Umsatzleistung erzielt, da auf der Mikroebene des erfindungsgemäßen Katalysators keine Transportlimitierung vorliegt. Durch die gleichzeitige Eliminierung des die Reaktion sonst limitierenden Wassers aus den Gleichgewichten der Gleichungen (1) und (2) durch die integrierte Wasserbindung kann das Gleichgewicht des Prozesses weiter auf die Produktseite verschoben werden. Dadurch ergeben sich Synergieeffekte und eine Effizienzsteigerung bei den Reaktionen (1) und (2). Auf diese Weise kann eine Verbesserung der Ausbeute erzielt werden. Darüber hinaus kann durch den Einsatz des erfindungsgemäßen Katalysators das Verfahren in für Umsatz und Selektivität günstigen Druck-und Temperaturbereichen durchgeführt werden.

Der Katalysator für das erfindungsgemäße Verfahren kann beispielsweise auf einfache und effiziente Weise dadurch hergestellt werden, dass der Fe-Katalysator-Anteil durch ein Vermischen entsprechender Metallsalze in Lösung, anschließender Fällung und Reduktion dargestellt und anschließend mit dem Zeolith-Anteil vermengt wird. Die Herstellung des Fe-Katalysator-Anteils durch eine Fällungsreaktion kann dabei in einem kontinuierlichen oder in einem Batchverfahren durchgeführt werden. Offenbart wird die Verwendung der Anlage zur Reduktion von CO₂-Emissionen von Kohlekraftwerken, Gaskraftwerken, Heizkraftwerken, Müllverbrennungsanlagen, Industrieanlagen, Schiffsaggregaten, Biogasanlagen, Pyrolyseanlagen und Verbrennungsmaschinen.

### Beschreibung der Figuren

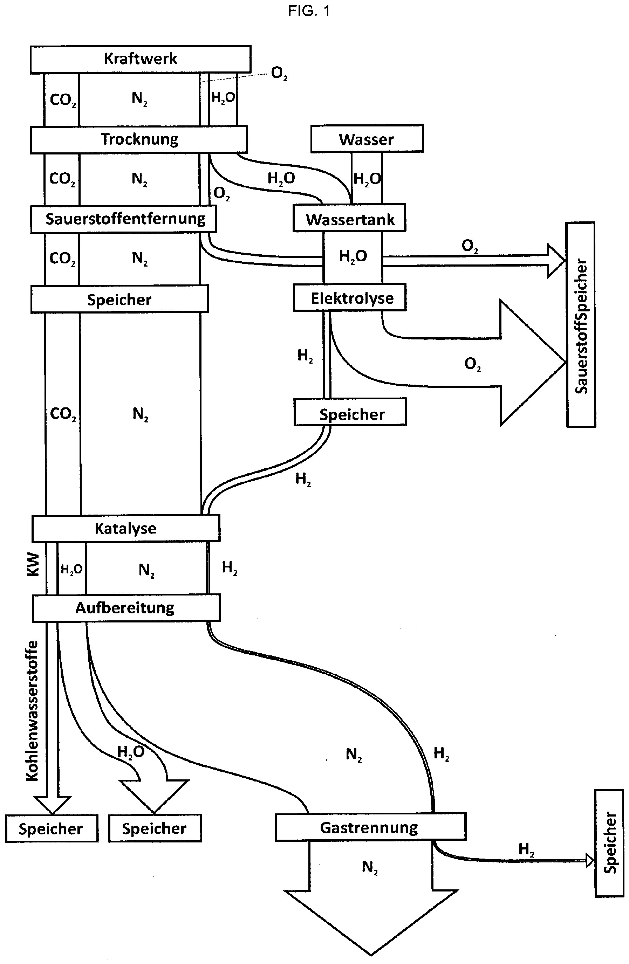

In Figur 1 sind in einem Stofffließbild beispielhaft die Massenströme des erfindungsgemäßen Verfahrens dargestellt. Als CO₂-Quelle dient hier ebenfalls beispielhaft der Abgasstrom eines kohlenstoffhaltige Brennstoffe verbrauchenden Kraftwerks. Der Abgasstrom enthält neben den bei der Verbrennung entstehenden CO₂ und Wasser zusätzlich Stickstoff und Sauerstoff. In einem Trocknungsschritt nach der Verbrennung im Kraftwerk wird dem Abgasstrom das Wasser entzogen, welches in einem Wassertank gesammelt werden kann. In einem weiteren Schritt kann dem Abgasstrom durch geeignete, dem Fachmann bekannte Verfahren und Anlagen der Sauerstoff entzogen werden und einem Sauerstoffspeicher zugeführt werden. Das dem Abgasstrom entzogene Wasser kann, im Bedarfsfall vermengt mit extern zugeführten zusätzlichen Wasser, durch Elektrolyse in Wasserstoff und Sauerstoff aufgetrennt werden. Der dafür benötigte elektrische Strom kann vorzugsweise durch Windkraft und/oder Wasserkraft gewonnen worden sein. So können beispielsweise Stromspitzen, die durch bestimmte Witterungslagen und/oder mangelnde Nachfrage im Stromnetz entstanden sind, verwendet bzw. aufgefangen werden. Dieses kann gerade im Fall von mangelnder Netzkapazität zum Abführen des erzeugten Stroms von erheblichen Vorteil sein. Der erzeugte Wasserstoff wird dann im erfindungsgemäßen Schritt der Katalyse mit dem erfindungsgemäßen Katalysator mit dem CO₂ und Stickstoff enthaltenden Gasstrom umgesetzt, so dass der Gasstrom nach der Katalyse neben Stickstoff, einem Rest Wasserstoff sowie die bei der Reaktion entstandenen Kohlenwasserstoffe sowie das ebenfalls entstandene Wasser umfasst. In einem anschließenden Aufbereitungsschritt werden in dem Fachmann bekannten Verfahren und Anlagen die Kohlenwasserstoffe sowie das Wasser aus dem Gasstrom entfernt und gespeichert. In einem letzten Schritt kann noch der eventuell im Gasstrom enthaltene Wasserstoff vom Stickstoff abgetrennt werden. Somit würde das beispielhaft dargestellte Kraftwerk (völliger Umsatz des bei der Verbrennung im Kraftwerk erzeugten CO₂ in der Katalyse zu den Kohlenwasserstoffen vorausgesetzt) lediglich Stickstoff emittieren. Mögliche Kreislaufschließungen sind in Figur 1 nicht dargestellt.

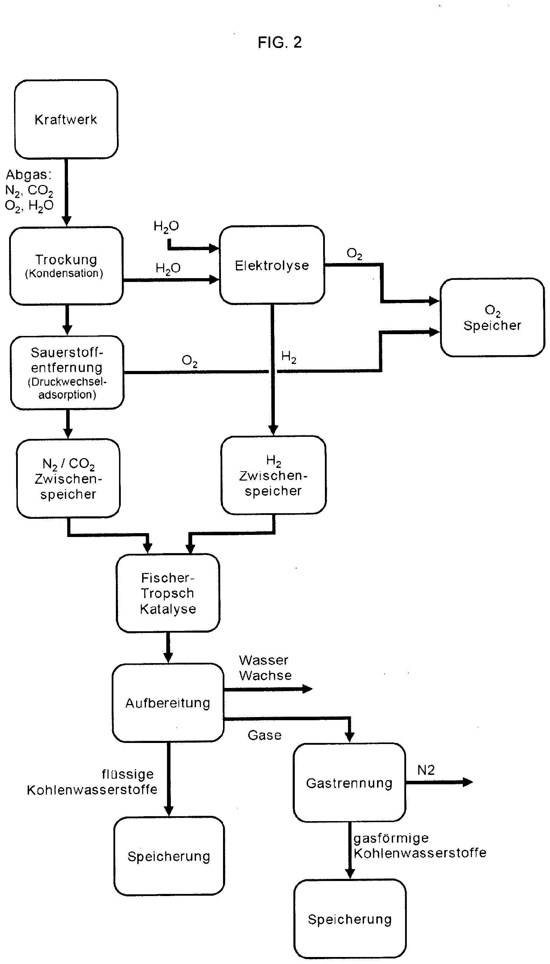

Das in Figur 1 beispielhaft als Fließbild gezeigte Konzept ist in Figur 2 in einer Variante als Blockschaltbild verdeutlicht. Hier ist die Trocknung beispielhaft als Kondensation und die Sauerstoffentfernung als Druckwechseladsorption ausgeführt. Im Aufbereitungsschritt nach der katalysierten Umsetzung des im Abgas enthaltenen CO₂ werden neben dem Wasser auch Kohlenwasserstoff-Wachse entfernt und ansonsten flüssige Kohlenwasserstoffe aufgefangen und dem Gasstrom später in einer Gastrennung auch vorhandene gasförmige Kohlenwasserstoffe abgetrennt. Somit werden in dem eingesetzten erfindungsgemäßen Verfahren flüssige, feste und gasförmige Kohlenwasserstoffe erhalten, die mittels dem Fachmann bekannter Techniken aus dem Abgasstrom der Katalysatoranlage gewonnen und abgetrennt werden können.

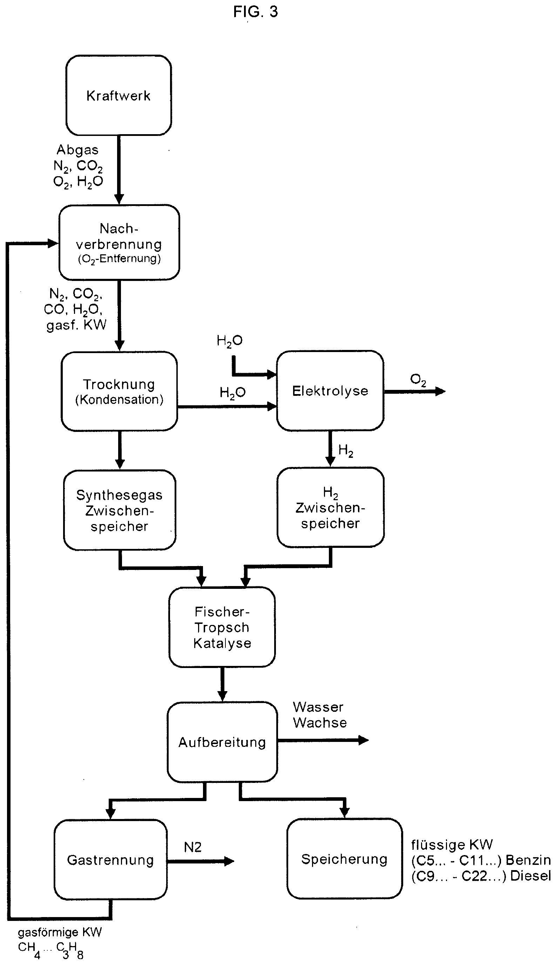

Figur 3 zeigt eine weitere Abwandlung des erfindungsgemäßen Verfahrens. Hier wird nun im Gegensatz zu der in Figur 2 dargestellten Ausführungsform der im Abgasstrom des Kraftwerks enthaltene Sauerstoff nicht mittels eines PSA-Verfahrens abgetrennt, sondern wird durch eine der Verbrennung im Kraftwerk nachgeschaltete Nachverbrennung verbraucht. Wie im Blockschaubild gezeigt wird, kann in einer Ausführungsform des erfindungsgemäßen Verfahrens zumindest ein Teil der im Verfahren erzeugten gasförmigen Kohlenwasserstoffe dazu verwendet werden, die noch im Abgasstrom vorhandenen Sauerstoffreste im Sinne einer Nachverbrennung zu verbrennen. Die dabei entstandene Wärme kann zu den im Kraftwerk eingesetzten Prozessen zur Wärme- und Stromgewinnung eingesetzt werden, die erzeugte Menge CO₂ wird erfindungsgemäß zusammen mit der schon im Kraftwerk bei einem Verbrennungsprozess erzeugten Menge CO₂ mit dem erfindungsgemäßen Katalysator und Wasserstoff zu Kohlenwasserstoffen umgesetzt.

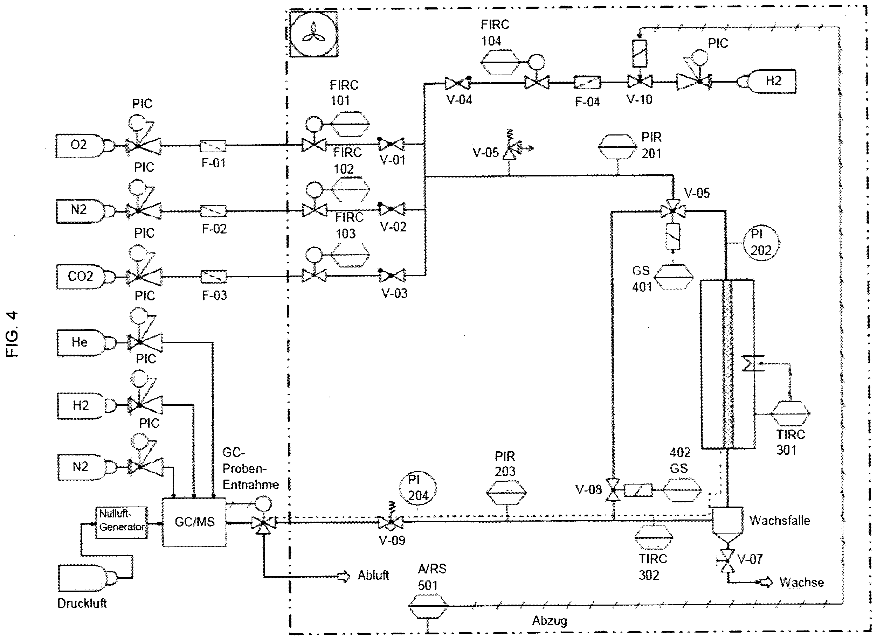

Figur 4 veranschaulicht ein Fließbild der Versuchsanlage, wie sie in den nachfolgend beschriebenen Beispielen der vorliegenden Erfindung verwendet wurde. Dabei sind die nachfolgend verwendeten Bauteile nicht einschränkend für die vorliegende Erfindung zu verstehen, sie beschreiben lediglich die Funktionsweise des ebenfalls rein beispielhaften Versuchsaufbaus. Im Wesentlichen besteht diese Anlage aus einer Gasmischstation zur Bereitstellung aller benötigten Gase, dem eigentlichen Synthesereaktor sowie der Produktanalytik in Form eines Gaschromatographen mit Massendetektor (GC-MS). Weiterhin ist die Versuchsanlage ausgestattet mit umfangreicher Mess-, Steuer- und Regelungstechnik zum automatisierten Betrieb der Anlage

Dabei haben die Nummerierungen und Abkürzungen im Fließbild die folgenden Bedeutungen:
- F-01, F-02, F-03, F-04:: Filter
- V-01, V-02, V-03, V-04:: Rückschlagventile
- V-05:: Überström-Sicherheitsventil
- V-06:: 3-Wege-Magnetventil
- V-07:: Handventil für Wachsfalle
- V-08:: 2-Wege-Magnetventil
- V-09:: Rückdruckventil
- V-10:: Magnetventil, stromlos geschlossen
- FIRC:: Massenflussregler
- PIC:: Druckminderer, analog
- PIR-201:: Druckaufnehmer
- PI-202:: Manometer, analog
- PIR-203:: Druckaufnehmer
- PI-204:: Manometer, analo
- TIRC-301:: Temperaturregelung Reaktor
- TIRC-302:: Temperaturregelung Begleitheizung
- GS-401:: Stellungssignal für Magnetventil
- GS-402:: Stellungssignal für Magnetventil
- AIRS-501:: Wasserstoff-Detektor

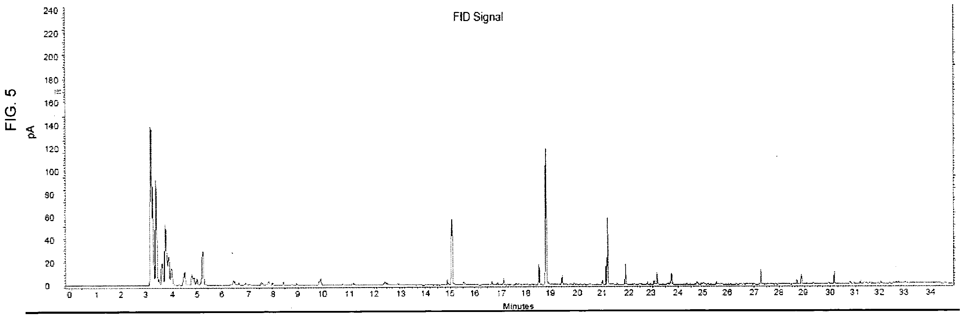

Figuren 5, 6 und 7 sind GC-MS-Spektren der Produktzusammensetzungen von der auf der Versuchsanlage wie hier beschrieben durchgeführten Beispielen und Vergleichsbeispielen. Dabei bezeichnet die x-Achse die Retentionszeit in Minuten und die y-Achse eine Spannung in der Einheit Pikoampere.

### Beispiele

Für alle im Folgenden betrachteten Versuchsreihen wurden folgende Gase als Synthesegas verwendet:

| | |
|---|---|
| Stickstoff | Qualität: 5.0 |
| Kohlendioxid | Qualität: 4.8 |
| Wasserstoff | Qualität: 5.0 |

Alle Gase wurden von der Firma Linde bezogen und in 10 Liter Gasdruckflaschen für den Betrieb der FTS-Laboranlage bereitgehalten. Der verwendete Zeolith HZSM-5 hatte ein Si/Al-Verhältnis von 10,8 welches durch den Hersteller Chemiewerk Bad Köstritz GmbH bestimmt wurde.

Als Versuchsanlage wurde eine Anlage verwendet, wie sie in Figur 4 dargestellt ist und beschrieben wurde.

Zur Bestimmung der Produktzusammensetzung der Versuchsanlage wurde der Produktstrom über eine beheizte Transferline (T = 170°C) aus der Laboranlage in das Injektionssystem des Gaschromatographen geleitet. Bei dem verwendeten Gaschromatographen handelte es sich um das Modell 7950 A der Firma Agilent, welches mit einem Wärmeleitfähigkeitsdetektor (WLD) und einem Flammenionisationsdetektor (FID) ausgestattet war. Mittels des Wärmeleitfähigkeitsdetektors wurden die im Produktstrom enthaltenen Permanentgase quantifiziert. Die entstehenden Kohlenwasserstoffe wurden mit dem Flammenionisationsdetektor quantifiziert und gleichzeitig mittels des Massendetektors 5975 C der Firma Agilent identifiziert. Der Gaschromatograph wurde durch die Firma SIM (Scientific Instruments Manufacturer GmbH) modifiziert, um eine direkte Injektion des Produktstroms zu gewährleisten und Kondensation von Produkten in den Rohrleitungen zu verhindern.

Für den Betrieb des Flammenionisationsdetektors wurde Wasserstoff der Qualität 5.0 sowie Druckluft (mit einer Reinigung durch einen Zero-Air-Generator der Firma Whatman) verwendet. Der Massendetektor wurde im Modus der Elektronenstoßionisation (EI) mit 70eV betrieben. Als Trägergas wurde Helium der Qualität 5.0 aus einer 50 Liter Druckgasflasche der Firma Linde verwendet. Als Druckminderer für die Heliumversorgung des Gaschromatographen wurde ein Flaschendruckminderer Modell F51M der Firma Elgas eingesetzt. Die Pneumatik zur Steuerung der Ventilschaltung des Injektionssystems des GCMS-Systems wurde mit Stickstoff betrieben. Zur Trennung der entstehenden Kohlenwasserstoffe wird eine CP-Sil PONA CB 50 x 0,21 (0,5) Säule der Firma Agilent verwendet. Zur Trennung der Permanentgase wird eine Stop-Flow-Schaltung mit Backflushfunktion verwendet, welches eine Schaltung aus drei Säulen darstellt. Verwendete Säulen zur Trennung der Permanentgase:

| | |
|---|---|
| - CP Sil PONA CB 15 x 0,32 (3,0) | Backflush |
| - PoraPLOT Q-HT 25 x 0,32 | 1. Trennung von Permanentgasen |
| - Molsieb 5A PLOT 30 x 0,32 (10) | Stop-Flow |

### Beispiel 1

### Herstellung des Eisenanteils b) des Katalysators in einem Batchverfahren

In einem Rundkolben wurden 60,6 g Fe(NO₃)₃ · 9 H₂O, 3,624 g Cu(NO₃)₂ · 3 H₂O und 7,315 g Al(NO₃)₃ · 9 H₂O in 250 ml Wasser gelöst. Es wurde auf 83 °C erwärmt. In einem weiteren Kolben wurden 250 ml einer 2,7 M Ammoniak-Lösung auf 83°C erwärmt. Zwei Schlauchpumpen wurden so angebracht, dass die vorgewärmten Lösungen gleichzeitig in den vorgewärmten Dreihalskolben gepumpt werden konnten. Der Dreihalskolben wurde dazu im Wasserbad erwärmt. Dabei wurde darauf geachtet, dass ein pH-Wert von 6 nicht überschritten wurde. Die Pumpgeschwindigkeit wurde daran angepasst. Anschließend wurde im Eisbad auf 0 °C zur Vervollständigung der Fällung gekühlt.

Es wurde über einen Büchnertrichter im Wasserstrahlvakuum filtriert (Schwarzbandfilter) und anschließend mit etwa 800 mL Wasser gewaschen. Danach wurde zunächst bei 50 °C für 48 h und anschließend für 12 h bei 120 °C getrocknet.

Entsprechend der erhaltenen Menge wurde mit K⁺ dotiert:
Es wurde eine der gewünschten Kupfermenge entsprechende Lösung von K₂CO₃ in möglichst wenig Wasser hergestellt. Diese wurde zu der getrockneten Eisenfällung gegeben. Dabei wurde darauf geachtet, dass die Lösung vollständig aufgenommen wurde. Anschließend wurde erneut für 12 h bei 120 C getrocknet.

Die Reduktion wurde vor dem Einsatz im Reaktor in einem Wasserstoffstrom (10% mit Stickstoff) durchgeführt.

Damit konnte auf eine effiziente Art und Weise der Eisenanteil b) des erfindungsgemäßen Katalysators erhalten werden.

### Beispiel 2

### Herstellung eines Katalysators der vorliegenden Erfindung

Ein erfindungsgemäßer Katalysator wurde dadurch hergestellt, dass der in Beispiel 1 hergestellte Eisenanteil b) im Verhältnis 1:6 mit Zeolith ZSM-5 vermengt wurde.

### Beispiel 3

Es wurde in der Anlage wie unter Figur 4 beschrieben ein wie in Beispielen 1 und 2 beispielhaft hergestellter Katalysator bis zu einer Füllhöhe von 10,9 cm in den Reaktor eingefüllt. Dabei wurden eine physikalische Mischung von 0,2 g des Metallkatalysators sowie 0,8 g an ZSM-5 Zeolith verwendet. Somit betrug das Mischungsverhältnis Zeolith zu Metallanteil 4:1.

Die Stöchiometrie von Wasserstoff zu CO₂ betrug 3:1. Das Verhältnis von CO₂ zu Stickstoff wurde entsprechend der Abgaszusammensetzung des von der Verbands-Energie-Werk Gesellschaft für Erneuerbare Energien mbH (VEW) betriebenen Biomassekraftwerkes in Bad Arolsen gewählt.

Der Fluss des aus Stickstoff, CO₂ und Wasserstoff bestehenden Synthesegases betrug 36 mlₙ min⁻¹. Daraus ergeben sich die Flüsse der einzelnen Gase in mlₙ min⁻¹, wie sie neben den anderen Versuchsbedingungen in Tabelle 1 zusammengefasst sind.

| | |
|---|---|
| n | 3 |
| CO₂ | 3,8 |
| H₂ | 11,4 |
| N₂ | 20,8 |
| Fluss gesamt | 36 |
| Temperatur T /°C | 320 |
| Druck /bar | 10 |
| Metallkatalysator /g | 0,2 |
| Zeolith (ZSM-5) /g | 0,8 |

Es wurde ein CO₂-Umsatz von 14 % beobachtet, d.h. es wurden 14 % des eingesetztem CO₂ in Kohlenwasserstoffe umgewandelt.

Ein GC-MS-Spektrum ist als Figur 5 angehängt. Darauf ist gut zu erkennen, dass neben kurzkettigen Kohlenwasserstoffen wie beispielsweise Ethan, Propan, Butan, Pentan insbesondere auch erhebliche Anteile an aromatischen Verbindungen wie Toluol, Xylol, Trimethylbenzol und Naphthaline erhalten werden.

Somit konnte gezeigt werden, dass mit dem erfindungsgemäßen Katalysator in dem erfindungsgemäßen Verfahren erfolgreich CO₂ in Kohlenwasserstoffe umgewandelt werden konnten und nicht nur die eigentlich zu erwartenden Fischer-Tropsch-Produkte, sondern auch insbesondere aromatische Verbindungen erhalten werden können.

### Beispiel 4

Es wurde analog Beispiel 3 verfahren, nur dass anstelle einer 4:1-Zeolith:Metallanteil-Mischung eine 8:1-Zeolith-Metallanteil-Mischung als Katalysator im Reaktor der Versuchsanlage verwendet wurde.

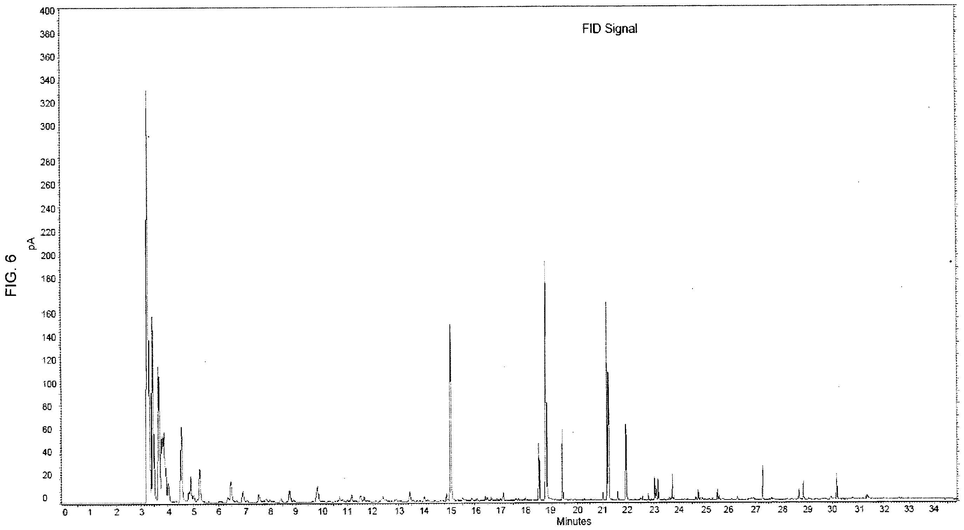

Dabei wurde ein CO₂-Umsatz von 23 % beobachtet, d.h. es wurden 23 % des eingesetztem CO₂ in Kohlenwasserstoffe umgewandelt. Das hier als Figur 6 beiliegende GC-MS-Spektrum ergab eine zu Beispiel 3 analoge Produktzusammensetzung, d.h. neben kurzkettigen Kohlenwasserstoffen wurden insbesondere auch aromatische Verbindungen erhalten.

### Vergleichsbeispiel 1

Es wurde analog der Beispiele 3 und 4 verfahren, allerdings wurde kein Zeolith der Katalysatormischung verwendet. Das bedeutet, dass ausschließlich Metallanteil gemäß Beispiel 1 verwendet wurde.

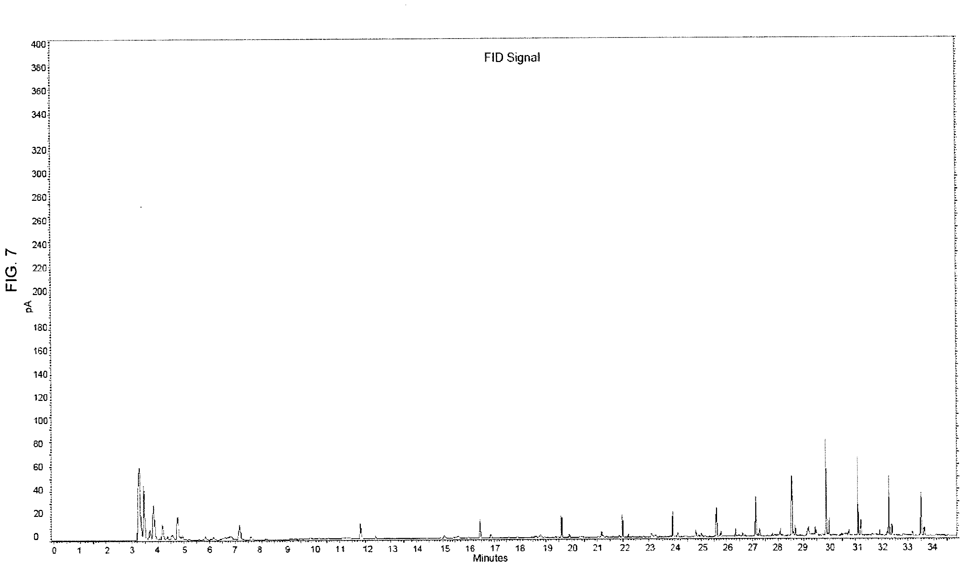

Es wurde gefunden, dass auf diese Weise lediglich ein CO₂-Umsatz von 8 % gefunden wurde. Darüber hinaus zeigte die hier als Figur 7 beiliegende GC-MS-Analyse der Produktzusammensetzung, dass es sich bei den gebildeten Produkten ausschließlich um gesättigte Kohlenwasserstoffe handelte. Im Bereich der aromatischen Kohlenwasserstoffe wurden im Gegensatz zu den Spektren der Beispiele 3 und 4 keine Produkte gefunden.

## Patentansprüche

1. Verfahren zur Umwandlung von CO₂ in flüssige Kohlenwasserstoffe, umfassend die Schritte
(i) Bereitstellen eines Gasstroms, umfassend CO₂ und Wasserstoffgas,
(ii) Kontaktieren des Gasstromes mit mindestens einem Katalysator, umfassend
(a) einen Zeolith-Anteil, und
(b) einen Fe-Katalysator-Anteil, umfassend Eisen im Bereich von 20 bis 200 Gewichtsteilen, einen Al₂O₃-Träger im Bereich von 5 bis 100 Gewichtsteilen, Kupfer im Bereich von 1 bis 40 Gewichtsteilen und mindestens ein Alkalimetall und/oder ein Erdalkalimetall im Bereich von 1 bis 40 Gewichtsteilen,
wobei die Anteile (a) und (b) in einer physikalischen Mischung in einem Verhältnis im Bereich von 10:1 bis 4:1 vorliegen,
bei einer Temperatur im Bereich von 250 bis 450 °C und einem Druck im Bereich von 0,1 bis 10 MPa,
dabei Umwandeln des CO₂ in Kohlenwasserstoffe.

2. Verfahren gemäß Anspruch 1, wobei der Gasstrom einen mit Wasserstoffgas angereicherten Abgasstrom eines Verbrennungsmotors, einer Turbine, einer Verbrennungsanlage, einer Industrieanlage, insbesondere Metallveredelungsanlage oder Härterei, und/oder eines Kraftwerks umfasst.

3. Verfahren gemäß Anspruch 1, wobei der Gasstrom ein Gasstrom aus einer Biogasanlage ist und zusätzlich CH₄ umfasst.

4. Verfahren gemäß Anspruch mindestens einem der Ansprüche 1 bis 3, wobei der Gasstrom zusätzlich ein Inertgas, bevorzugt ausgewählt aus der Gruppe bestehend aus Stickstoff, Helium, Neon, Argon sowie Gemischen daraus, besonders bevorzugt Stickstoff, enthält.

5. Verfahren gemäß einem der vorangehenden Ansprüche, wobei das Alkalimetall Kalium ist.

6. Verfahren gemäß einem der vorangehenden Ansprüche, weiter umfassend einen Schritt (ia) Erzeugen von Wasserstoffgas durch Elektrolyse.

7. Verfahren gemäß Anspruch 5 wobei die Elektrolyse (ia) mittels durch Wasserkraft und/oder Windkraft und/oder einer Biogasanlage und/oder einer Pyrolseabfallanlage erzeugter Elektrizität durchgeführt wird.

8. Verfahren gemäß einem der vorangehenden Ansprüche, wobei der Schritt (ii) bei einer Temperatur im Bereich von 300 bis 400 °C und einem Druck im Bereich von 0,5 bis 8 MPa, bevorzugt im Bereich von 0,8 bis 4 MPa durchgeführt wird.

9. Verfahren gemäß einem der vorangehenden Ansprüche, wobei der Katalysator in Form eines Wirbelbettes, eines Festbettes und /oder einer Beschichtung eingesetzt wird.

10. Verfahren gemäß einem der vorangehenden Ansprüche, wobei der Schritt (ii) in einem mikrostrukturierten Reaktor durchgeführt wird.

11. Verfahren gemäß einem der vorangehenden Ansprüche, wobei der Fe-Katalysator-Anteil b) Eisen im Bereich von 40 bis 180 Gewichtsteilen, bevorzugt 70 bis 150 Gewichtsteilen, Al₂O₃ im Bereich von 10 bis 90 Gewichtsteilen, bevorzugt im Bereich von 20 bis 70 Gewichtsteilen, Kupfer im Bereich von 5 bis 30 Gewichtsteilen, bevorzugt im Bereich von 8 bis 20 Gewichtsteilen und mindestens ein Alkalimetall und/oder ein Erdalkalimetall im Bereich von 5 bis 30 Gewichtsteilen, bevorzugt im Bereich von 8 bis 20 Gewichtsteilen umfasst.

12. Verfahren gemäß einem der vorangehenden Ansprüche, wobei der Zeolith des Zeolith-Anteils des Katalysators ein Pentasil-Zeolith, bevorzugt ZSM-5, ist.

## Claims

1. A method for the conversion of CO₂ into liquid hydrocarbons, comprising the steps of:
(i) providing a gas stream comprising CO₂ and hydrogen gas,
(ii) contacting the gas stream with at least one catalyst comprising
(a) a zeolite component, and
(b) a Fe catalyst component, comprising iron in the range of 20 to 200 parts by weight, an Al₂O₃ carrier in the range of 5 to 100 parts by weight, copper in the range of 1 to 40 parts by weight, and at least one alkali metal and/or alkaline earth metal in the range of 1 to 40 parts by weight,
wherein in a physical mixture the components (a) and (b) exist in a ratio in the range from 10:1 to 4:1,
at a temperature in the range of 250 to 450 °C and at a pressure in the range of 0.1 to 10 MPa,
thereby converting the CO₂ into hydrocarbons.

2. The method according to claim 1, wherein the gas stream comprises a hydrogen gas-enriched exhaust stream of a combustion engine, a turbine, a combustion plant, an industrial plant, in particular a metal refining plant or a hardening plant, and/or a power plant.

3. The method according to claim 1, wherein the gas stream is a gas stream from a biogas plant and additionally comprises CH₄.

4. The method according to at least one of claims 1 to 3, wherein the gas stream additionally contains an inert gas, preferably selected from the group consisting of nitrogen, helium, neon, argon and mixtures thereof, more preferably nitrogen.

5. The method according to any of the preceding claims, wherein the alkali metal is potassium.

6. The method according to any of the preceding claims, further comprising the step (ia) generating hydrogen gas by means of electrolysis.

7. The method according to claim 5, wherein the electrolysis (ia) is carried out by means of electricity generated by hydroelectric force and/or wind power and/or a biogas plant and/or a pyrolysis waste plant.

8. The method according to any of the preceding claims, wherein step (ii) is carried out at a temperature in the range from 300 to 400 ° C and at a pressure in the range from 0.5 to 8 MPa, preferably in the range from 0.8 to 4 MPa.

9. The method according to any of the preceding claims, wherein the catalyst is used in the form of a fluidized bed, a fixed bed and/or a coating.

10. The method according to any of the preceding claims, wherein step (ii) is carried out in a microstructured reactor.

11. The method according to any of the preceding claims, wherein the Fe catalyst component (b) comprises iron in the range from 40 to 180 parts by weight, preferably from 70 to 150 parts by weight, Al₂O₃ in the range from 10 to 90 parts by weight, preferably in the range from 20 to 70 parts by weight, Copper in the range from 5 to 30 parts by weight, preferably in the range from 8 to 20 parts by weight, and at least one alkali metal and/or an alkaline earth metal in the range from 5 to 30 parts by weight, preferably in the range from 8 to 20 parts by weight.

12. The method according to any of the preceding claims, wherein the zeolite of the zeolite component of the catalyst is a pentasil zeolite, preferably ZSM-5.

## Revendications

1. Procédé destiné à transformer du CO₂ en hydrocarbures liquides, comprenant les étapes
(i) de la mise à disposition d'un flux gazeux, comprenant du CO₂ et du gaz d'hydrogène,
(ii) de la mise en contact du flux gazeux avec au moins un catalyseur, comprenant
(a) une part de zéolithe et
(b) une part de catalyseur Fe, comprenant du fer dans l'ordre de 20 à 200 parts en poids, un porteur Al₂O₃ dans l'ordre de 5 à 100 parts en poids, du cuivre dans l'ordre de 1 à 40 parts en poids et au moins un métal alcalin et/ou un métal alcalino-terreux dans l'ordre de 1 à 40 parts en poids,
les parts (a) et (b) se présentant dans un mélange physique, dans une proportion de l'ordre de 10 : 1 à 4 : 1,
à une température de l'ordre de 250 à 450 ° et sous une pression de l'ordre de 0,1 à 10 MPa,
à cet effet, transformation du CO2 en hydrocarbures.

2. Procédé selon la revendication 1, le flux gazeux comprenant un flux de gaz d'échappement enrichi de gaz d'hydrogène d'un moteur thermique, d'une turbine, d'une station d'incinération, d'une installation industrielle, notamment d'une installation d'affinage des métaux ou d'un atelier de trempe et/ou d'une centrale électrique.

3. Procédé selon la revendication 1, le flux gazeux comprenant un flux gazeux issu d'une installation de biogaz et comprenant en plus du CH₄.

4. Procédé selon au moins l'une quelconque des revendications 1 à 3, le flux gazeux contenant en plus un gaz inerte, choisi de préférence dans le groupe consistante en l'azote, l'hélium, le néon, l'argon, ainsi que des mélanges de ceux-ci, de manière particulièrement préférée, de l'azote.

5. Procédé selon l'une quelconque des revendications précédentes, le métal alcalin étant du potassium.

6. Procédé selon l'une quelconque des revendications précédentes, comprenant en outre une étape (ia) de la génération de gaz d'hydrogène par électrolyse.

7. Procédé selon la revendication 5, l'électrolyse (ia) étant réalisée au moyen d'électricité générée par énergie hydraulique, par énergie éolienne et/ou par une installation de biogaz et/ou par une station de traitement de déchets par pyrolyse.

8. Procédé selon l'une quelconque des revendications précédentes, l'étape (ii) étant réalisée à une température de l'ordre de 300 à 400 °C et sous une pression de l'ordre de 0,5 à 8 MPa, de préférence dans l'ordre de 0,8 à 4 MPa.

9. Procédé selon l'une quelconque des revendications précédentes, le catalyseur étant mis en oeuvre sous la forme d'un lit fluidisé, d'un lit fixe et/ou d'un revêtement.

10. Procédé selon l'une quelconque des revendications précédentes, l'étape (ii) étant réalisée dans un réacteur microstructuré.

11. Procédé selon l'une quelconque des revendications précédentes, la part en catalyseur Fe b) comprenant du fer dans l'ordre de 40 à 180 parts en poids, de préférence de 70 à 150 parts en poids, de l'Al₂O₃ dans l'ordre de 10 à 90 parts en poids, de préférence dans l'ordre de 20 à 70 parts en poids, du cuivre dans l'ordre de 5 à 30 parts en poids, de préférence dans l'ordre de 8 à 20 parts en poids et au moins un métal alcalin et/ou un métal alcalino-terreux dans l'ordre de 5 à 30 parts en poids, de préférence dans l'ordre de 8 à 20 parts en poids.

12. Procédé selon l'une quelconque des revendications précédentes, la zéolithe de la part de zéolithe du catalyseur étant une zéolithe de type pentasile, de préférence du ZSM-5.
